Europäisches Patentamt

European Patent Office

Office européen des brevets

(19)

(11) **EP 1 418 398 A1**

(12) **EUROPEAN PATENT APPLICATION**
published in accordance with Art. 158(3) EPC

(43) Date of publication:
**12.05.2004 Bulletin 2004/20**

(51) Int Cl.⁷: **G01B 11/24**

(21) Application number: **02746070.8**

(22) Date of filing: **15.07.2002**

(86) International application number:
**PCT/JP2002/007186**

(87) International publication number:
**WO 2003/008904 (30.01.2003 Gazette 2003/05)**

(84) Designated Contracting States:
**AT BE BG CH CY CZ DE DK EE ES FI FR GB GR
IE IT LI LU MC NL PT SE SK TR**

(30) Priority: **17.07.2001 JP 2001217309**

(71) Applicant: **Sanyo Electric Co., Ltd.
Moriguchi City, Osaka 570-8677 (JP)**

(72) Inventors:
• **FUJITA, Hideto c/o Sanyo Electric Co., Ltd.
Moriguchi City, Osaka 570-8677 (JP)**

• **KANO, Hiroshi c/o Sanyo Electric Co., Ltd.
Moriguchi City, Osaka 570-8677 (JP)**
• **TEJIMA, Masayuki c/o Sanyo Electric Co., Ltd.
Moriguchi City, Osaka 570-8677 (JP)**
• **YOSHIDA, Hiroaki c/o Sanyo Electric Co., Ltd.
Moriguchi City, Osaka 570-8677 (JP)**
• **FUKUMOTO, Shinpei c/o Sanyo Electric Co., Ltd.
Moriguchi City, Osaka 570-8677 (JP)**

(74) Representative: **Glawe. Delfs. Moll
Patentanwälte
Postfach 26 01 62
80058 München (DE)**

(54) **SHAPE MEASURING DEVICE**

(57)    In a shape measuring device comprising a measuring head for measuring the shape of an object to be measured at a plurality of measuring positions on a guide rail while moving along the guide rail, the guide rail is in a U shape, and the measuring head makes measurement for measuring the shape of the object to be measured which is arranged inside the U-shaped guide rail at the plurality of measuring positions on the guide rail while moving along the guide rail.

FIG. 1

**Description**

Technical Field

The present invention relates to a shape measuring device comprising a measuring head for measuring the shape of an object to be measured at a plurality of measuring positions on a guide rail while moving along the guide rail, and more particularly, to a shape measuring device suited to measure the shape of a foot.

Background Art

Conventionally, an active stereo-type shape measuring device that irradiates a beam of spot tight, slit light, or the like onto an object to be measured and restores from the position of a light image observed on a surface of the object to be measured its three-dimensional shape has been known. An example of such a shape measuring device is one that scans spot light or slit light by a rotary mirror in order to measure the shape of an object to be measured. A magazine "Measurement and Control" (1999 Vol. 38 No. 4 P285-288) describes a system for measuring the shape of a foot using such a shape measuring device.

In the above-mentioned system for measuring the shape of a foot, one shape measuring device can measure only the shape of a portion observed from the device and cannot measure the shape of a portion concealed on the opposite side thereof, for example. Therefore, 12 shape measuring devices are arranged around the foot, and the results of the measurement by the 12 shape measuring devices are synthesized on a computer, thereby measuring the shape of the whole of the foot.

In this system, however, the plurality of shape measuring devices are arranged around the foot. Therefore, not only the problem that the system increases in size and increases in cost but also the problem that it is difficult to synthesize the results of measurement by the plurality of shape measuring devices with high precision arises.

On the other hand, a shape measuring device that measures the shape of the whole of an object to be measured by a measuring person gripping a measuring head and moving the measuring head around the object to be measured to make measurement. In the shape measuring device, a plurality of markers attached to the measuring head are imaged from above by two cameras, thereby measuring the position and the direction of the measuring head.

In a case where the shape of a human foot is measured, it is considered that a guide rail in an elliptical shape (in such a shape that two sides extending along the lengths of opposed two semicircles are connected to each other) of such a size as to enclose the human foot is provided, and a measuring head is moved along the guide rail.

When the guide rail for moving the measuring head is made to have a loop shape such as a circular shape or an elliptical. shape, as described above, the size of an object to be measured whose shape can be measured is limited by the size of the guide rail. That is, in a case where the object to be measured is even slightly larger than a measurable range including the moving margins of the guide rail and the measuring head, the shape cannot be measured. Particularly, in a case where the shape of a foot is measured, the size of the foot differs among individuals, and differs depending on races. For example, the feet of the Westerner are larger than those of the Japanese. Therefore, the foot may not, in some cases, be arranged inside the guide rail in a loop shape.

The present invention has been made in view of such a problem and has for its object to provide a shape measuring device capable of making the size of an object to be measured which can be measured have a degree of freedom. When a human foot is measured, for example, an object is to provide a shape measuring device that can measure the shape of the foot irrespective of the difference in the size of the foot depending on adults or children, sex, races, or the like.

Disclosure of Invention

In a shape measuring device comprising a measuring head for measuring the shape of an object to be measured at a plurality of measuring positions on a guide rail while moving along the guide rail, a shape measuring device according to the present invention is characterized in that the guide rail is in a U shape, and the measuring head makes measurement for measuring the shape of the object to be measured which is arranged inside the U-shaped guide rail at the plurality of measuring positions on the guide rail while moving along the guide rail.

In a case where the object to be measured is a human foot, the direction in which the foot is arranged is determined such that. a direction from inside a curved portion of the U-shaped guide rail toward an opened portion thereof is a direction along the length of the foot. In this case, the measuring head makes measurement for measuring the shape of a foot arranged inside the U-shaped guide rail at the plurality of measuring positions on the guide rail while moving along the guide rail.

In a case where the object to be measured is a human foot, used as the measuring head is one comprising a light source for irradiating a light beam toward the foot arranged inside the U-shaped guide rail and a camera for imaging the light beam irradiated onto the foot by the light source. The coordinates of the light beam imaged by the camera are calculated at the plurality of measuring positions on the U-shaped guide rail so that the shape of the foot arranged inside the guide rail is measured.

Brief Description of Drawings

Fig. 1 is a schematic view showing the configuration of a shape measuring device according to an embodiment of the present invention.
Fig. 2 is a plan view showing a measuring head.
Fig. 3 is a partially cutaway plan view showing the configuration of a measuring head.
Fig. 4 is an explanatory view for explaining the principle of measurement.
Fig. 5 is an explanatory view for explaining a method of measuring the position of a measuring point using a measuring head.
Fig. 6 is a schematic plan view showing the positional relationship among a foot serving as an object to be measured, a guide rail, and a measuring head.
Fig. 7 is a flow chart showing the procedure for control processing of a measuring head by a control device.
Fig. 8 is a schematic view showing the displacement of a measuring head.
Fig. 9 is a plan view showing a modified example of a guide rail.

Best Mode for Carrying Out the Invention

Description is now made of an embodiment of the present invention.

[1] Description of Shape Measuring Device

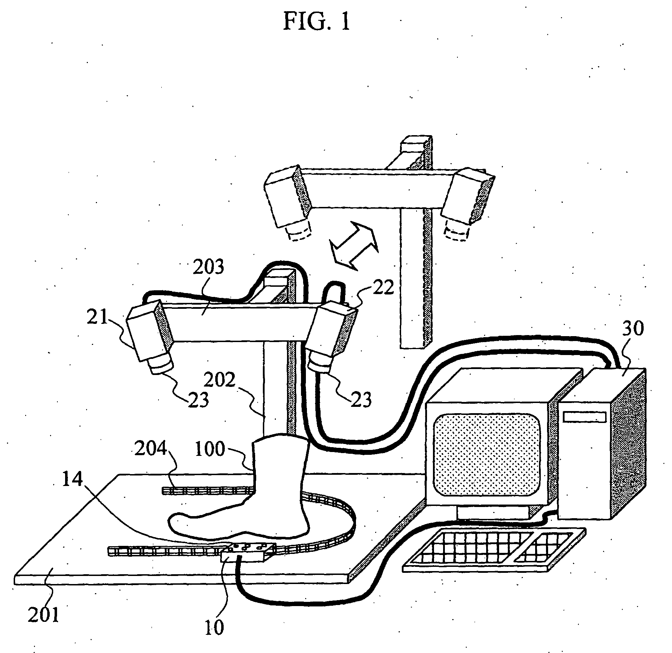

Fig. 1 illustrates the schematic configuration of a shape measuring device according to an embodiment of the present invention.

A U-shaped guide rail 204 is fixed to a measuring stand 201, and a foot 100 serving as an object to be measured is put on an area enclosed by the guide rail 204.

Furthermore, a support 202 which is detachable from the stand 201 is attached to the stand 201, and a horizontal bar 203 is attached to its upper part.

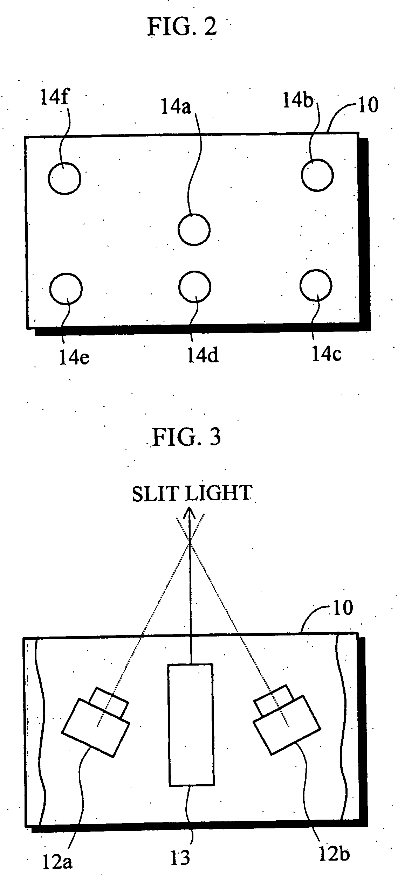

The shape measuring device comprises a measuring head 10 which is moved on the guide rail 204 by a measuring person, stereo cameras 21 and 22 attached to both ends of the horizontal bar 203, and a control device 30 composed of a personal computer for carrying out their control, various types of operations, and the like. A band-pass filter 23 which selectively transmits frequency bands of light beams emitted by six markers 14 shown in Fig. 2 is attached to an imaging lens of each of the stereo cameras 21 and 22.

Figs. 2 and 3 illustrate the schematic configuration of the measuring head 10.

The measuring head 10 comprises a casing in a rectangular parallelepiped shape and opened forward, two CCD cameras 12a and 12b accommodated in the casing, and a slight light source 13 so constructed that laser light is scanned upward and downward to form slit light. Further, the measuring head 10 comprises a driving mechanism (not shown) for moving in a clockwise direction or in a counterclockwise direction along the guide rail 204.

Furthermore, the marker 14 comprising six LED light sources 14a and 14f is provided on an upper surface of the casing of the measuring head 10.

The arrangement of the six LED light sources constituting the marker 14 is not point-symmetrical but line-symmetrical with respect to the center line of the measuring head 10 in order to specify the direction of the measuring head 10. Here, the five LED light sources 14b, 14c, 14d, 14e, and 14f are arranged so as to take a rectangle on the upper surface of the casing, and the one LED light source 14a is arranged at the position of its center of gravity.

Although at least three LED light sources may be sufficient as the marker 14 in order to measure the position and the direction of the measuring head 10 in a three-dimensional space, four or more LED light sources are used to improve the measuring precision of the position and the direction of the measuring head 10 in a least square manner.

The measuring head 10 is attached so as to be movable along the guide rail 204 by a supporting mechanism (not shown). Further, the measuring head 10 comprises an encoder (not shown) for detecting the position of the measuring head 10 using as a basis a predetermined position on the guide rail 204. An output of the encoder 10 is inputted to the control device 30.

[2] Description of Principle of Measurement of Shape Measuring Device

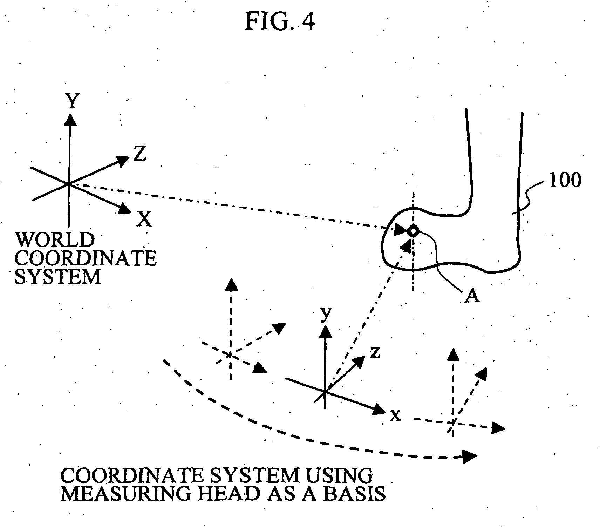

The basic principle of measurement of the shape measuring device will be described on the basis of Fig. 4.

The coordinates of a certain measuring point A are measured using the measuring head 10 which is moved on the guide rail 204 by the measuring person. The measured coordinates are represented by coordinates (x, y, z) in a coordinate system using a measuring head as a basis (centered with respect to a measuring head). The coordinate system is a coordinate system which moves as the measuring head 10 moves.

On the other hand, the shape of the object to be measured 100 is represented by a fixed coordinate system, and the coordinate system is referred to as a world coordinate system. The coordinates, in the world coordinate system, of a measuring point measured by the measuring head 10 are taken as (X, Y, Z). The shape of the object to be measured 100 must be described by the world coordinate system. Accordingly, the coordinates (x, y, z), in the coordinate system using a measuring head as a basis, of the measuring point A measured by the measuring head 10 are converted into the coordinates (X, Y, Z) in the world coordinate system. The conversion is performed on the basis of the following equation (1) using a rotating matrix R and a translation vector t which represent the movement of the measuring head 10:

$$
\begin{vmatrix} X \\ Y \\ Z \end{vmatrix} = R \cdot \begin{vmatrix} x \\ y \\ z \end{vmatrix} + t \qquad \dots (1)
$$

Consequently, the coordinates (x, y, z) in the coordinate system using a measuring head as a basis can be converted into the coordinates (X, Y, Z) in the world coordinate system by finding the position and the direction of the measuring head 10 in the world coordinate system as the rotating matrix R and the translation vector t.

[3] Description of Procedure for Shape Measurement Processing by Shape Measuring Device

The measurement of the shape by the shape measuring device is made by the following procedure for processing.

First, preliminary processing (the following first and second steps) is performed before making actual measurement.

[3-1] First Step

Specifically, information related to each or measuring positions of the measuring head 10 in the world coordinate system is stored in a memory (not shown) carried on the control device 30 in correspondence with the output value of the encoder at the measuring position. Information related to each of the measuring positions of the measuring head 10 in the world coordinate system is the rotating matrix R and the translation vector t which represent the movement of the measuring head 10 in the world coordinate system.

Description is made more concretely.

The coordinates, in the world coordinate system, of each of the markers 14 provided in the measuring head 10 are measured by the stereo cameras 21 and 22. Since the position measuring method has been well known as a stereo method, the description thereof is omitted.

The coordinates, in the coordinate system using a measuring head as a basis, of each of the LED light sources 14a to 14f constituting the marker 14 are then respectively taken as $(x_i, y_i, z_i)$, and the coordinates, in the world coordinate system, of each of the LED light sources 14a to 14f measured by the stereo cameras 21 and 22 are respectively taken as $(X_i, Y_i, Z_i)$, where i is 1, 2, ... 6. The coordinates $(x_i, y_i, z_i)$, in the coordinate system using a measuring head as a basis, of each of the LED light sources 14a to 14f have already been known.

The rotating matrix R and the translation vector t which represent the movement of the measuring head 10 are found as a matrix R and a vector t which satisfy the following equation (2). The found matrix R and vector t are stored in the memory in correspondence with the output value of the encoder at the measuring position of the measuring head 10.

$$min \sum_i [(Xi - xi)^2 + (Yi - yi)^2 + (Zi - zi)^2 \,]$$

$$here \qquad\qquad ...\,(2)$$

$$\begin{vmatrix} X_i \\ Y_i \\ Z_i \end{vmatrix} = R \cdot \begin{vmatrix} x_i \\ y_i \\ z_i \end{vmatrix} + t$$

The measuring head 10 is moved along the guide rail 204, and the above-mentioned processing is repeatedly performed with respect to all the measuring positions on the guide vail 204, to produce a table representing a correspondence between the output value of the encoder and the rotating matrix and the translation vector t at the position.

[3-2] Second Step

The support 202 for supporting the stereo cameras 21 and 22 is removed from the stand 201. In such a manner, the object to be measured 100 is actually measured after the preliminary processing is completed. Description is now made of a case where the object to be measured is a human foot 100.

[3-3] Third Step

The coordinates of a measuring point on the object to be measured (foot) 100 in the coordinate system using a measuring head as a basis are found using the measuring head 10.

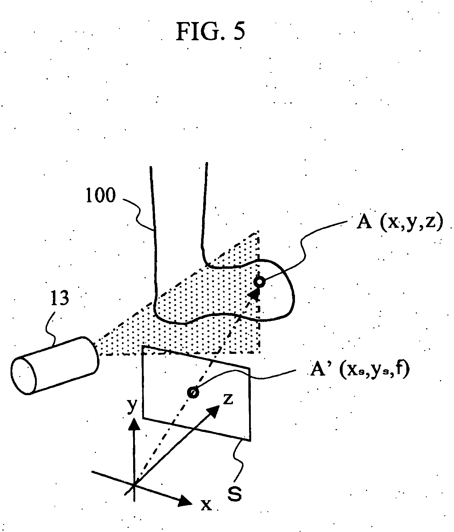

Fig. 5 illustrates a method of measuring the position of a measuring point by the measuring head 10.
As shown in Fig. 5, the coordinate system using a measuring head as a basis is a coordinate system having the optical center of the CCD camera 12 as its origin, having the direction of the optical axis as its z-axis, the horizontal direction of the CCD camera 12 as its x-axis, and the vertical direction of the CCD camera 12 as its y-axis. An image surface S of the CCD camera 12 exists at a position spaced a focal length f apart from the origin. That is, the image surface S is a plane parallel to an x-y plane and satisfying z = f.
A method of measuring a position by the measuring head 10 is itself a known measuring method called a light-section method. A predetermined point on a line irradiated with the slit light from the slit light source 13 on a surface of the object to be measured 100 is taken as a measuring point A.
The coordinates, in the coordinate system using a measuring head as a basis, of the measuring point A are taken as (x, y, z), the coordinates of an observing point A' corresponding to the measuring point A on the image surface S are taken as (xs, ys, f), and an equation of a plane representing the slit light is taken as ax + by + cx + d = 0. In the coordinates (xs, ys, f) of the observing point A', f has already been known as the focal length of the CCD camera 12, and (xs, ys) are found from the pixel position of the slit light observed on the image surface.
The equation of the plane representing the slit light is found by the calibration of the measuring head 10. Consequently, a simultaneous equation expressed by the following equation (3) having x, y, z, and as unknowns is solved, thereby finding (x, y, z) :

$$ax + by + cz + d = 0$$
$$x = \alpha \cdot x_s$$
$$y = \alpha \cdot y_s \qquad ...\,(3)$$
$$z = \alpha \cdot f$$

The processing is performed by the control device 30 on the basis of an output of the CCD camera 12.

[3-4] Fourth Step

In the fourth step, the rotating matrix R and the translation vector t which correspond to each other are read out of the memory in the control device 30 on the basis of the output of the encoder.

[3-5] Fifth Step

In the fifth step, the coordinates (x, y, z), in the coordinate system using a measuring head as a basis, which is found in the third step, of the measuring point on the object to be measured (foot) 100 are converted into the coordinates (X, Y, Z) in the world coordinate system on the basis of the rotating matrix R and the translation vector t which are read out in the fourth step.

The measuring head 10 is moved along the. guide rail 204, and the processing in the third to fifth steps is repeated with respect to all the measuring positions on the guide rail 204, thereby finding the shape of the object to be measured (foot) 100 as a set of the coordinates (X, Y, Z), in the world coordinate system, of the measuring point obtained each time.

As described in the foregoing, according to the present embodiment, the coordinates, in the coordinate system using a measuring head as a basis, of the measuring point obtained using the measuring head 10 are converted into the coordinates in the world coordinate system using table data representing a correspondence between the position on the guide rail 204 of the measuring head 10 and the rotating matrix R and the translation vector t at the position. Accordingly, the necessity of the stereo cameras 21 and 22 is eliminated in making shape measurement, and a user can measure the shape of the object to be measured without being conscious of the field of view of a.camera and the tangle of a code, thereby making it possible to provide higher usability.

[4] Description of Method of Controlling Measuring Head 10

Description is made of a method of controlling the measuring head 10 by the control device 300 in the embodiment using the U-shaped guide rail 204.

At the time of measurement, the foot is placed inside the guide rail 204. In this case, the foot is placed with the central position of a circular arc portion of the. U-shaped guide rail 204 taken as the center of its heel. To thus place the foot makes it possible to measure, with respect to feet of various sizes (various types of feet having different lengths), the respective shapes of the feet.

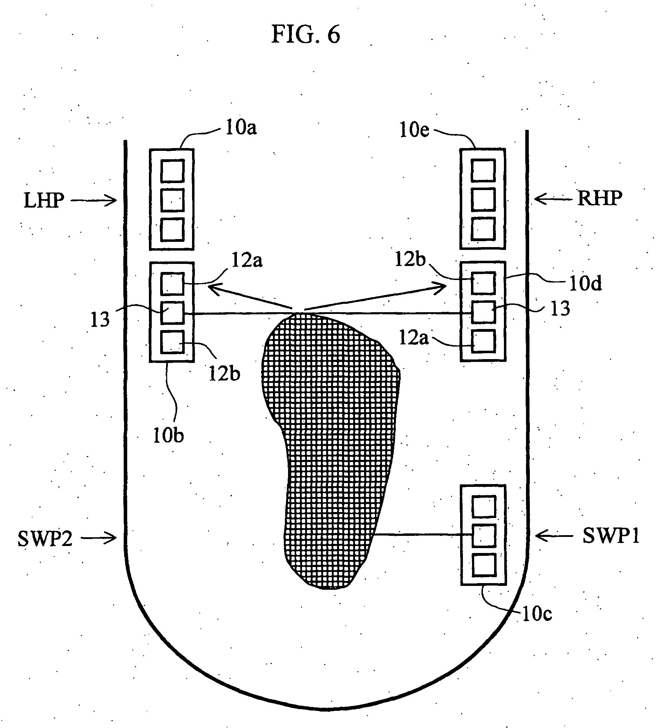

Fig. 6 illustrates in a time series manner the movement of the measuring head 10 in a case where the right foot 100 is placed inside the U-shaped guide rail 204 to measure the shape of the right foot.

10a indicates a case where the measuring head 10 is at a left home position LHP. When the measuring head 10 is at the left home position LHP, the measuring head 10 is moved in a counterclockwise direction along the guide rail 204 from the left home position LHP.

10b indicates a case where the measuring head 10 is at the position where the toe of the right foot 100 is detected. 10c indicates a case where the measuring head 10 is at a camera switching position for a right foot SWP1 set near the right ankle of the right foot 100. 10d indicates a case where the measuring head 10 is at the position where the measurement is terminated, which is calculated from the position where the toe is detected, indicated by 10a. 10e indicates a case where the measuring head 10 is at a right home position RHP.

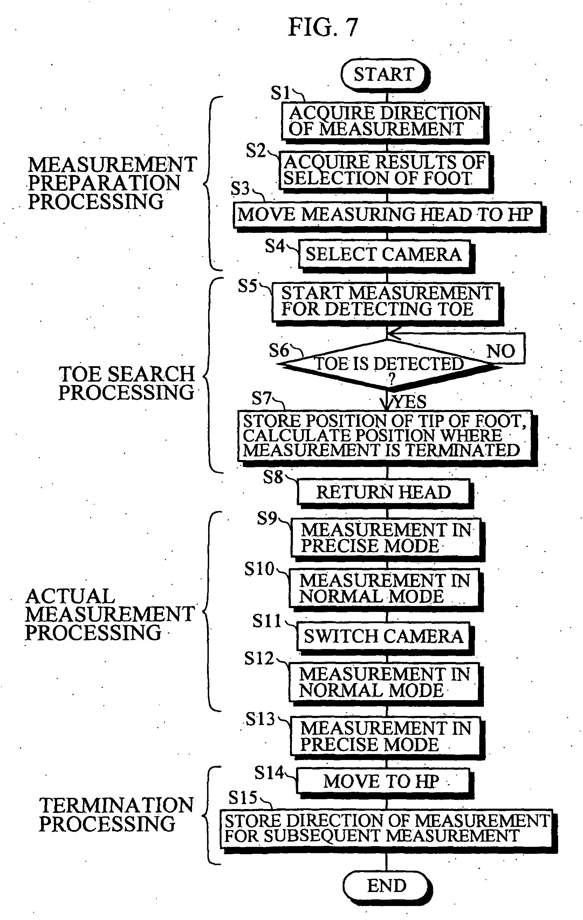

Fig. 7 shows the procedure for control processing of the measuring head 10 by the control device 300.

First, processing in the steps S1 to S4 is performed as measurement preparation processing.

In the measurement preparation processing, the direction of measurement is first acquired (step S1). That is, the direction of movement of the measuring head 10 (a clockwise direction or a counterclockwise direction) is acquired. When the foot has already been measured, the direction of subsequent measurement is stored in the final step S15, described later, so that the stored direction of subsequent measurement is acquired as the current direction of measurement. When the direction of subsequent measurement is not stored, the direction of measurement in which the movement is started is automatically selected on the basis of a home position near the current position of the measuring head 10.

That is, the counterclockwise direction is selected as the direction of measurement when the measuring head 10 is at a position near the left home position LHP, while the clockwise direction is selected as the direction of measurement when the measuring head 10 is at a position near the right home position RHP. In this example, it is assumed that the measuring head 10 is at the position near the left home position LHP, and the counterclockwise direction is acquired as the direction of measurement.

The results of selection as to whether the foot to be measured is a right foot or a left foot is then acquired (step S2). Selection as to whether the foot to be measured is a right foot or a left foot is made by an operator performing a predetermined selection operation on a display with respect to the control device 300. In this example, it is assumed that the right foot is selected, as in the example shown in Fig. 6.

The measuring head 10 is then moved to the home position (HP) determined by the direction of measurement (step S3). That is, the measuring head 10 is moved to the left home position LHP (the position indicated by 10a in Fig. 6) when the direction of measurement is the counterclockwise direction, while being moved to the right home position RHP (the position indicated by 10e in Fig. 6) when the direction of measurement is the clockwise direction. In this example, it is assumed that the measuring head 10 is moved to the left home position LHP because the direction of measurement is the counterclockwise direction.

Thereafter, the camera first used for the measurement is selected on the basis of the direction of measurement (step S4). That is, out of the two cameras 12a and 12b, the camera positioned on the rear side in the direction of measurement is selected. The camera 12a is selected when the direction of movement is the counterclockwise direction, while the camera 12b is selected when the direction of measurement is the clockwise direction.

In this example, it is assumed that the camera 12a is selected as the camera first used for measurement. Consequently, the camera which more easily detects the toe, that is, the camera 12a positioned on the rear side in the direction of movement is selected as the camera first used for measurement, as indicated by 10b in Fig. 6.

When the measurement preparation processing in the Steps S1 to S4 is terminated, toe search processing in the steps S5 to S7 is performed. The toe search processing is processing for detecting the toe of the foot. In the toe search processing, shape measurement is not made.

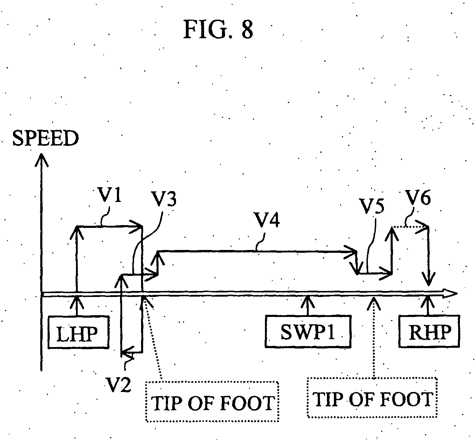

In the toe search processing, measurement for detecting the toe (the tip of the foot) is first started by increasing the movement speed of the measuring head 10 (step S5). That is, measurement for detecting the toe is made while moving the measuring head 10 at high speed, as indicated by an arrow V1 in Fig. 8. When the toe is detected (step S6), the measuring head 10 is stopped, to store in the memory the position of the detected toe (the position of the tip of the foot) as well as to calculate the position where the measurement is terminated from the position of the detected toe and store the calculated position in the memory (step S7).

In this example, the measuring head leads to the position indicated by 10b in Fig. 6, so that the position of the toe is detected. From the position of the detected toe, the position of a right-hand portion of the guide rail 204 (the position indicated by 10d in Fig. 6) corresponding thereto is calculated as the position where the measurement is terminated.

In the toe search processing, the measuring head 10 is moved at high speed, so that the measuring head 10 goes beyond (overruns) the position corresponding to the position of the tip of the foot at the time point where the tip of the foot is found. Therefore, the measuring head 10 is returned by a predetermined distance (approximately 3 to 5 cm) at high speed (step S8). In this example, the measuring head 10 is moved by a predetermined distance in a clockwise direction, as indicated by an arrow V2 in Fig. 8.

Actual measurement processing in the steps S9 to S12 will be then performed. In this measurement processing, measurement processing for measuring the shape of the foot is performed. In this measurement processing, measurement processing in a precise mode is first performed (step S9). That is, measurement processing is performed while moving the measuring head 10 at low speed and with the spacing between the measuring positions reduced. In this example, the measurement processing is performed while moving the measuring head 10 at low speed and with the spacing between the measuring positions reduced, as indicated by an arrow V3 in Fig. 8.

The reason why the measurement processing in a precise mode is thus performed in the vicinity of an opened end of the guide rail 204 is that the measurement precision is made lower because the measurement is made by the camera arranged beside the toe of the foot in the vicinity of the opened end of the guide rail 204, as compared with that at a curved portion of the guide rail 204, so that the spacing between the measuring positions is reduced to heighten the measurement precision.

When the measuring head 10 goes beyond a range set to approximately 5 cm, for example, from the position of the toe, measurement processing in a normal mode is performed (step S10). That is, after the measuring head 10 passes through the vicinity of the toe, measurement processing is performed while moving the measuring head 10 at intermediate speed and with the spacing between the measuring positions increased. In this example, the measurement processing is performed while moving the measuring head 10 at intermediate speed and with the spacing between the measuring positions increased, as indicated by an arrow V4 in Fig. 8.

The spacing between the measuring positions in the measurement processing in a normal mode is set to approximately 1.5 times the spacing between the measuring positions in the measurement processing in a precise mode. The range in which the measurement processing in a precise mode in the step S9 is performed can be previously set in the control device 30 or automatically set on the basis of the size of the foot.

When the measuring head 10 reaches a predetermined camera switching position while the measurement processing in a normal mode in the step S10 is being performed, the camera used for the measurement is switched (step S11). In a case where the right foot is measured, when the measuring head 10 reaches the camera switching position for a right foot SWP1 (see Fig. 6) set in the vicinity of the right ankle irrespective of the direction of measurement, the camera used for the measurement is switched. In a case where the left foot is measured, when the measuring head 10 reaches the camera switching position for a left foot SWP2 (see Fig. 6) set in the vicinity of the left ankle irrespective of the direction of measurement, the camera used for the measurement is switched.

In this example, the right foot is measured, and the camera first used for the measurement is the camera 12a. When the measuring head 10 reaches the camera switching position for a right foot SWP1 shown in Fig. 6, therefore, the camera used for the measurement is switched from the first camera 12a to the second camera 12b.

Even after the camera used for the measurement is switched, the measurement processing in a normal mode is performed (step S12).

When the camera used for the measurement is switched, the position of the camera used for the measurement is changed, so that operation processing for shape measurement is also switched. The camera used for the measurement is switched, so that the effect of a measuring error is small because there are relatively few irregularities at the position of the ankle of the foot even if there occurs such inconvenience that an error or discontinuity slightly occurs in the operation processing for shape measurement.

Thereafter, when the measuring head 10 reaches a position 5 cm ahead of the toe, measurement processing in a precise mode is performed (step S13). In this example, the measurement processing is performed while moving the measuring head 10 at low speed and with the spacing between the measuring positions reduced, as indicated by an arrow V5 in Fig. 8.

It is judged whether or not the measuring head 10 reaches the position 5 cm ahead of the toe by taking the position where the measurement is terminated (the position indicated by 10d in Fig. 6 in this example), which is calculated in the step S7, as the position of the toe. The measurement processing in the step S13 is continued until the measuring head 10 reaches the position where the measurement is terminated (the position indicated by 10d in Fig. 6 in this example) or a position a predetermined distance (e.g., approximately 2cm) beyond the position.

As described in the foregoing, when the measurement processing for measuring the shape of the right foot is terminated, the measuring head is moved at high speed to the home position determined by the direction of measurement (step S14). When the direction of measurement is the counterclockwise direction as in this example, the measuring head 10 is moved to the right home position RHP, as indicated by an arrow V6 in Fig. 8. When the direction of measurement is the clockwise direction, the measuring head 10 is moved to the left home position LHP.

Thereafter, a direction opposite to the direction of current measurement is stored in the memory as the direction of subsequent measurement (step S15). The current processing for controlling the measuring head 10 is terminated.

According to the above-mentioned embodiment, the guide rail is in a U shape. Accordingly, the size of the object to be measured and particularly, the length of the object to be measured can be made to have a degree of freedom, as compared with a shape measuring device having a guide rail in a loop shape such as a circular shape or an elliptical shape.

[5] Description of Modified Example

Although in the above-mentioned embodiment, the foot is arranged such that the toe of the foot is directed toward the opened end of the U-shaped guide rail 204 and the heel of the foot is positioned on the side of the curved portion of the guide rail 204, the foot may be arranged such that the heel is directed toward the opened end of the U-shaped guide rail 204 and the toe is positioned on the side of the curved portion of the guide rail 204. This makes it possible to increase the measuring precision of the toe.

Although in the above-mentioned embodiment, information related to the position of the measuring head in the world coordinate system, that is, the rotating matrix R and the translation vector t are found using the outputs of the stereo cameras 21 and 22 , the rotating matrix R and the translation vector t can be found without using the stereo cameras 21 and 22 if the track of the guide rail 204 with respect to the stand 201 is previously specified.

Although description was made of the embodiment using the U-shaped guide rail 204, an embodiment utilizing an elliptical guide rail 204 which is sufficiently long in the longitudinal direction is within the range of the right of the present invention in a case where the measuring head 10 is moved in one of circular arc portions of the guide rail 204 so as to measure the heel, while not being moved in the other circular arc portion, that is, a case where the guide rail 204 is used as a substantially U-shaped guide rail.

For example, when the shape of the foot of an adult is measured, the whole of the elliptical guide rail may be utilized, to make shape measurement of such a system that a measuring head goes around the whole of the guide rail. On the other hand, when the shape of the foot of a child is measured, a part of the elliptical guide rail may be utilized as the U-shaped guide rail in the above-mentioned embodiment by arranging the foot in such a manner that the heel of the foot is positioned at the central position of one of circular arcs of the guide rail.

Furthermore, used as the measuring head 10 may be one different from that in the above-mentioned embodiment, provided that it measures the position of a measuring point on an object to be measured by an active stereo measuring method. For example, the slit light source 13 may be replaced with a spot light source. Although used as the above-mentioned measuring head 10 is one comprising two sets of measurement optical systems, that is, two CCD cameras 12a and 12b and one slit light source 13, a measuring head comprising one set of measurement optical systems, that is , a measuring head comprising a CCD camera and one slit light source may be used.

Although used as the U-shaped guide rail is one comprising a semicircular curved portion and two straight line portions respectively extending from both ends thereof, a guide rail which differs in shape from that shown in Fig. 6 may be used, provided that it is in an approximately U shape.

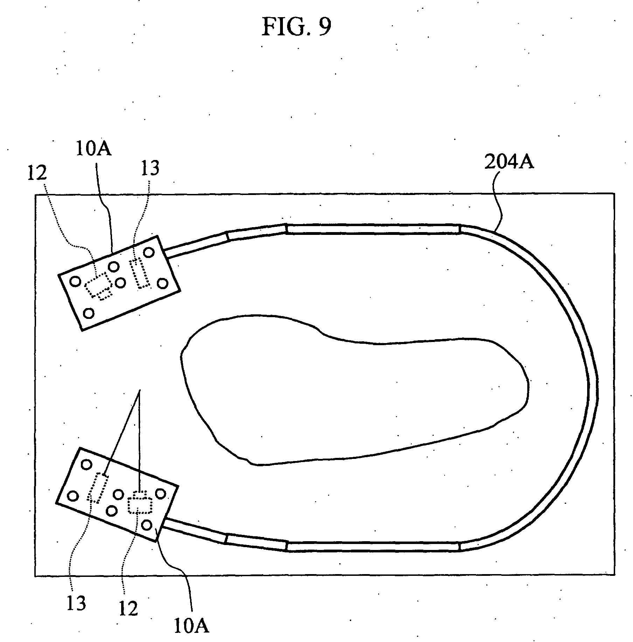

In order to enlarge the measurable range of the opened end, for example, a guide rail 204A in such a shape that an opened end in a U shape is narrowed down inward may be used, as shown in Fig. 9. As shown in Fig. 9 , even in a case where a measuring head 10A comprising one CCD camera 12 and one slit light source 11, that is, comprising one set of measurement optical systems is used, it is found that the measurable range in the vicinity of the opened end of the guide rail 204A is enlarged.

**Claims**

1. In a shape measuring device comprising a measuring head for measuring the shape of an object to be measured at a plurality of measuring positions on a guide rail while moving along the guide rail,

   a shape measuring device **characterized in that** the guide rail is in a U shape, and the measuring head makes measurement for measuring the shape of the object to be measured which is arranged inside the U-shaped guide rail at the plurality of measuring positions on the guide rail while moving along the guide rail.

2. The shape measuring device according to claim 1, **characterized in that** the object to be measured is a human foot, and the direction in which the foot is arranged is determined such that a direction from inside a curved portion of the U-shaped guide rail toward an opened portion thereof is a direction along the length of the foot.

3. The shape measuring device according to claim 2, **characterized in that** the measuring head makes measurement for measuring the shape of a foot arranged inside the U-shaped guide rail at the plurality of measuring positions on the guide rail while moving along the guide rail.

4. The shape measuring device according to claim 3, **characterized in that** the measuring head comprises a light source for irradiating a light beam toward the foot arranged inside the U-shaped guide rail and a camera for imaging the light beam irradiated onto the foot by the light source, the coordinates of the light beam imaged by the camera being calculated at the plurality of measuring positions on the U-shaped guide rail so that the shape of the foot arranged inside the guide rail is measured.

FIG. 1

FIG. 2

14f       14a       14b   10

14e       14d       14c

FIG. 3

SLIT LIGHT

10

12a       13       12b

FIG. 4

WORLD COORDINATE SYSTEM

100

A

COORDINATE SYSTEM USING
MEASURING HEAD AS A BASIS

FIG. 5

100

13

A (x,y,z)

A' ($x_s$,$y_s$,f)

y

z

x

S

FIG. 6

## FIG. 7

START

MEASUREMENT PREPARATION PROCESSING

- S1 — ACQUIRE DIRECTION OF MEASUREMENT
- S2 — ACQUIRE RESULTS OF SELECTION OF FOOT
- S3 — MOVE MEASURING HEAD TO HP
- S4 — SELECT CAMERA

TOE SEARCH PROCESSING

- S5 — START MEASUREMENT FOR DETECTING TOE
- S6 — TOE IS DETECTED ? — NO
  - YES
- S7 — STORE POSITION OF TIP OF FOOT, CALCULATE POSITION WHERE MEASUREMENT IS TERMINATED

S8 — RETURN HEAD

ACTUAL MEASUREMENT PROCESSING

- S9 — MEASUREMENT IN PRECISE MODE
- S10 — MEASUREMENT IN NORMAL MODE
- S11 — SWITCH CAMERA
- S12 — MEASUREMENT IN NORMAL MODE
- S13 — MEASUREMENT IN PRECISE MODE

TERMINATION PROCESSING

- S14 — MOVE TO HP
- S15 — STORE DIRECTION OF MEASUREMENT FOR SUBSEQUENT MEASUREMENT

END

FIG. 8

FIG. 9

## INTERNATIONAL SEARCH REPORT

| International application No. |
| --- |
| PCT/JP02/07186 |

**A. CLASSIFICATION OF SUBJECT MATTER**
Int.Cl⁷ G01B11/24

According to International Patent Classification (IPC) or to both national classification and IPC

**B. FIELDS SEARCHED**

Minimum documentation searched (classification system followed by classification symbols)
Int.Cl⁷ G01B11/00-11/30, A43B1/02, A61B5/10

Documentation searched other than minimum documentation to the extent that such documents are included in the fields searched
| | | | |
| --- | --- | --- | --- |
| Jitsuyo Shinan Koho | 1926-1996 | Jitsuyo Shinan Toroku Koho | 1996-2002 |
| Kokai Jitsuyo Shinan Koho | 1971-2002 | Toroku Jitsuyo Shinan Koho | 1994-2002 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y | JP 2001-41723 A (Sanyo Electric Co., Ltd.), 16 February, 2001 (16.02.01), Full text; all drawings (Family: none) | 1-4 |
| Y | JP 6-249626 A (Kenji MIMURA), 09 September, 1994 (09.09.94), Full text; all drawings (Family: none) | 1-4 |
| A | JP 9-14930 A (Matsushita Electric Industrial Co., Ltd.), 17 January, 1997 (17.01.97), Full text; all drawings (Family: none) | 1-4 |

☐ Further documents are listed in the continuation of Box C.   ☐ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 11 October, 2002 (11.10.02) | 29 October, 2002 (29.10.02) |

| Name and mailing address of the ISA/ | Authorized officer |
| --- | --- |
| Japanese Patent Office | |
| Facsimile No. | Telephone No. |

Form PCT/ISA/210 (second sheet) (July 1998)